(19) Europäisches Patentamt

European Patent Office

Office européen des brevets

(11) **EP 4 599 771 A1**

(12) **EUROPEAN PATENT APPLICATION**

(43) Date of publication:
**13.08.2025 Bulletin 2025/33**

(21) Application number: **24156086.1**

(22) Date of filing: **06.02.2024**

(51) International Patent Classification (IPC):
**A61B 8/00** *(2006.01)*    **A61B 8/08** *(2006.01)*

(52) Cooperative Patent Classification (CPC):
**A61B 8/461; A61B 8/467; A61B 8/5207;**
A61B 8/0866; A61B 8/0883

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Koninklijke Philips N.V.
5656 AG Eindhoven (NL)**

(72) Inventors:
• **KOTHAWALA, Aliarshad Aameer
Eindhoven (NL)**

• **MATTUR, Shashaank Aswatha
Eindhoven (NL)**
• **VAYYETI, Anudeep
5656AG Eindhoven (NL)**
• **SETH, Subhendu
Eindhoven (NL)**
• **VAJINEPALLI, Pallavi
Eindhoven (NL)**

(74) Representative: **Philips Intellectual Property &
Standards
High Tech Campus 52
5656 AG Eindhoven (NL)**

(54) **DEFINING IMAGING PARAMETERS OF A MEDICAL IMAGING SYSTEM**

(57) A mechanism for defining imaging parameters or characteristics of a medical imaging system. Medical imaging data, produced by the medical imaging system, is analyzed to generate a value for each of one or more image quality metrics. In particular, a different image quality estimation algorithm is used to generate the value for each, respective image quality metric. The determined values are used to define or control the imaging parameters of the medical imaging system.

FIG. 2

**Description**

FIELD OF THE INVENTION

The present invention relates to the field of medical imaging, and in particular, to defining parameters for performing medical imaging.

BACKGROUND OF THE INVENTION

There is an ongoing interest in the field of medicine for using medical imaging techniques to analyze and/or assess the condition of a subject or patient. In particular, there is a need for reliable and accurate generation of medical imaging data of a subject

A wide variety of medical imaging system are known, such as those that employ ultrasound imaging, magnetic resonance (MR) imaging, computed tomography (CT) imaging, position emission tomography (PET) imaging and so on.

There is a demand for medical imaging data of high quality. This is essential for a clinician or individual to assess the medical imaging data to determine one or more conclusions, e.g., about a condition or pathology of the subject. There is therefore a clear need for high quality data acquisition and generation of medical imaging data.

SUMMARY OF THE INVENTION

The invention is defined by the claims.

According to examples in accordance with an aspect of the invention, there is provided a computer-implemented method for defining and/or tuning imaging parameters of a medical imaging system.

The computer-implemented method comprises: obtaining, from the medical imaging system, medical imaging data of a subject; processing the medical imaging data using two or more image quality estimation algorithms to produce a measured value for each of a respective two or more image quality metrics; obtaining a desired quality indicator indicating a desired value for each of the two or more image quality metrics; and defining imaging parameters of the medical imaging system responsive to the measured value and the desired value for each of the two or more image quality metrics.

The present disclosure provides a technique for tuning medical imaging parameters of a medical imaging system responsive to two or more quality metrics. In particular, two or more different algorithms are used to generate a respective measure for each of a respective two or more different quality metrics. The values generated in this way are used to control or define the imaging parameters of the medical imaging system (e.g., for future imaging).

The proposed technique thereby provides a mechanism for quality-based tuning of the imaging parameters. Two or more measures of different quality metrics are used to perform the tuning of the imaging parameters.

In some examples, the (step of) defining imaging parameters comprises: defining a weighting for each of the two or more image quality metrics responsive to the desired quality indicator; and defining imaging parameters responsive to the weighting and measured value for each of the two or more image quality metrics.

In this way, different types of quality metric may be controllably prioritized (i.e., weighted). This can be exploited to facilitate a user control over the desired qualities of the medical imaging data or to configure the desired qualities to a particular use case scenario. It is recognized that different types of quality metric or image quality attributes may be favored over others depending upon the circumstances. For instance, in assessing a liver of a subject, then high and well developed speckle imaging data may be preferred over extreme contrast (but low speckle) imaging data. As another example, in assessing the spine of a subject, high contrast and good resolution imaging data may be preferable over high speckle (but low contrast/resolution) imaging data.

The use of weightings of image quality metrics (e.g., as loss functions), allows for balancing between different types of quality metric to be performed. This provides greater flexibility in selecting or defining the imaging parameters for achieving more flexible and/or controllable imaging outcomes. More particularly, a greater range and choice of imaging outcomes and quality parameters can be achieved.

The obtaining the desired quality indicator may comprise obtaining the desired quality indicator from a user input provided at a user input interface. This facilitates user control over the quality metric(s) of medical imaging data.

The (step of) obtaining the desired quality indicator may comprise: obtaining an anatomical region indicator that indicates an anatomical region; and processing the anatomical region indicator using a look-up table, which cross-references anatomical region indicators to desired quality indicators, to obtain the desired quality indicator. This provides an anatomy-specific mechanism for prioritizing or targeting particular image qualities of medical imaging data.

The obtaining the anatomical region indicator may comprise processing the medical imaging data, using an object detection algorithm, to identify the spatial location of anatomical region represented in the medical imaging data.

The medical imaging data may comprise ultrasound imaging data. In some examples, the medical imaging data comprises one or more 2D ultrasound images.

The two or more image quality metrics may, in some examples, comprise at least two of the following: a speckle quality metric; a spatial or intensity contrast metric; and/or a resolution metric.

Each image quality estimation algorithm may comprise: processing the medical imaging data using one or more Fourier decomposition techniques, to produce a spatial frequency representation of the medical imaging data; dividing the spatial frequency representation into at least two different frequency ranges to form a respective at least two partial spatial frequency representations; and processing each partial spatial frequency using a different image quality estimation algorithm to produce the measured value for each of a respective two or more image quality metrics.

Dividing the spatial frequency representation may comprise: obtaining an anatomical indicator that indicates an anatomical region; and defining the at least two different frequency ranges responsive to the anatomical indicator.

The computer-implemented method may further comprise controlling a user output interface to provide a visual representation of the measured value of each of the two or more image quality metrics.

There is also provided a computer program product comprising computer program code means which, when executed on a computing device having a processing system, cause the processing system to perform all of the steps of any herein disclosed method.

There is also provided a processing system for defining imaging parameters of a medical imaging system.

The processing system is configured to: obtain, from the medical imaging system, medical imaging data of a subject; process the medical imaging data using two or more image quality estimation algorithms to produce a measured value for each of a respective two or more image quality metrics; obtain a desired quality indicator indicating a desired value for each of the two or more image quality metrics; and define imaging parameters of the medical imaging system responsive to the measured value and the desired value for each of the two or more image quality metrics.

The processing system may be configured to define imaging parameters by: defining, for image quality metric, a weighting for the image quality metric responsive to the desired quality indicator; and defining imaging parameters responsive to the weighting and measured value for each of the two or more image quality metrics.

There is also provided a system comprising: the processing system previously described; and an ultrasound imaging system, configured to function as the medical imaging system, wherein the ultrasound imaging system comprises an ultrasound probe configured to generate ultrasound imaging data of the subject to function as the medical imaging data of the subject.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 illustrates a system in which embodiments may be employed;
Fig. 2 is a flowchart illustrating a proposed method;
Fig. 3 is a flowchart illustrating a step for use in the proposed method;
Fig. 4 is a flowchart illustrating another step for use in the proposed method;
Fig. 5 illustrates techniques for generating a value for different image quality metrics;
Fig. 6 illustrates a further proposed method; and
Fig. 7 illustrates a visual representation provided by the further proposed method.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for defining imaging parameters or characteristics of a medical imaging system. Medical imaging data, produced by the medical imaging system, is analyzed to generate a value for each of one or more image quality metrics. In particular, a different image quality estimation algorithm is used to generate the value for each, respective image quality metric. Each image quality metric captures or quantifies a different aspect of image quality such as contrast, resolution, texture or speckle quality, and so on. The determined values are used to define or control the imaging parameters of the medical imaging system.

Embodiments are based on the realization that different imaging parameters will prioritize or influence different types of image quality metric. Thus, imaging parameters that are optimized or designed for one type of image quality metric may have a negative impact on other types of image quality metric. The present disclosure provides a technique in which multiple image quality metrics are taken into account in setting or defining the imaging parameters of the medical imaging system.

Further embodiments provide techniques for weighting or prioritizing different types of image quality metric, e.g., over other image quality metrics. This advantageously allows the imaging parameters to be tuned to a compromise position for different image quality metrics, e.g., designed for use with a particular clinician and/or anatomical feature/-subject.

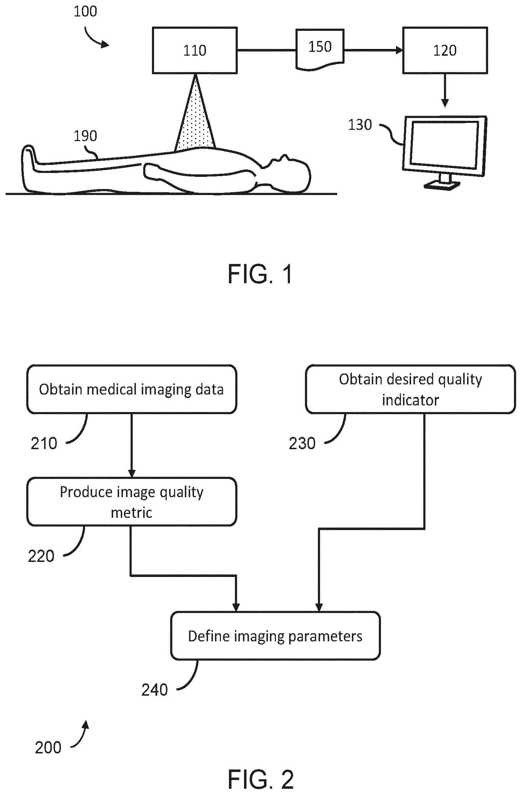

Fig. 1 illustrates a system 100 in which proposed embodiments may be employed. The system 100 comprises a medical imaging system 110 and a processing system 120.

The medical imaging system 110 is configured to capture and/or generate medical imaging data 150 of a subject 190. Examples of suitable medical imaging systems 110 are well known in the art, and include ultrasound imaging systems (e.g., comprising an ultrasound probe), magnetic resonance imaging (MRI) systems, computer tomography (CT) imaging systems, X-ray imaging systems, positron emission tomography (PET) imaging systems and so on.

It will be appreciated that imaging parameters are used by the medical imaging system 110 in the capturing and/or generation of the medical imaging data 150. The imaging parameters may, for instance, include one or more image acquisition parameters, image sampling parameters and/or image processing parameters. Image acquisition parameters may include parameters of the medical imaging system that define properties of any radiation, electric fields, magnetic fields and/or (ultra)sound produced during an imaging process. Image sampling parameters may include parameters of the medical imaging system used during sampling of raw data or medical imaging data (e.g., a pixel sensitivity, thresholds for sensing and so on). Image processing parameters may include any parameters of the medical imaging system for performing processing on captured or sampled data to produce the medical imaging data, e.g., type of processing technique used and/or parameters within any processing technique.

The skilled person will readily appreciate that different forms of medical imaging system will have different imaging parameters that can be defined, adjusted and/or modified. Alternative labels for imaging parameters include imaging settings. Examples include any adjustable value that affects how imaging data is obtained and/or subsequently processed.

The processing system 120 is configured to receive the medical imaging data 150 produced by the medical imaging system and perform further (image) processing on the medical imaging data 150. Thus, the processing system 120 is configured to perform one or more further processing tasks on the medical imaging data 150.

Existing examples of processing systems are capable of a variety of image processing tasks, such as image segmentation, object recognition, image enhancement, noise filtering, and so on.

Some processing systems may be configured to control a display 130 for providing a visual representation of the (e.g., processed) medical imaging data. The display 130 may form part of the system 100.

The present disclosure provides a mechanism for configuring a processing system to perform a task of controlling or defining one or more imaging parameters of the medical imaging system responsive to medical imaging data obtained by the medical imaging system.

In this way, the processing system is able to function as a feedback system for tuning the generation of medical imaging data by the processing system. This can advantageously be exploited to produce medical imaging data having desirable or desired characteristics, e.g., characteristics demanded by a clinician and/or best suited for a particular purpose of imaging and/or a particular anatomical structure that has been imaged.

Fig. 2 is a flowchart illustrating a computer-implemented method 200 for defining imaging parameters of a medical imaging system. The computer-implemented method may be carried out by the processing system previously described.

The computer-implemented method 200 comprises a step 210 of obtaining, from the medical imaging system, medical imaging data of a subject.

Step 210 may comprise receiving the medical imaging data of the subject or retrieving the medical imaging data of the subject (e.g., pulling the medical imaging data from the medical imaging system). In some examples, step 210 is performed using a memory or storage unit (e.g., a buffer) as an intermediary between the medical imaging system and the processing system. Thus, step 210 may comprise obtaining, from a memory or storage unit in communication with medical imaging system, the medical imaging data of a subject. Of course, this memory or storage unit may be considered to form part of the medical imaging system.

As a working example, the medical imaging data may comprise ultrasound imaging data. For instance, the medical imaging data may comprise one or more 2D ultrasound images.

The computer-implemented method 200 also comprises a step 220 of processing the medical imaging data using two or more image quality estimation algorithms to produce a measured value for each of a respective two or more image quality metrics. Thus, each image quality metric is associated with a different image quality estimation algorithm.

A number of example image quality estimation algorithms are described later in this disclosure. By way of working example, suitable image quality metrics that may be employed in the proposed approach include: a speckle quality metric; a spatial or intensity contrast metric; and/or a resolution metric.

The computer-implemented method 200 also comprises a step 230 of obtaining a desired quality indicator indicating a desired value for each of the two or more image quality metrics.

In a simple example, the desired quality indicator may be predefined. Thus, each desired value may be predefined.

In another example, the desired quality indicator may be defined by a user input provided at a user interface. Thus, step 230 may comprise obtaining the desired quality indicator from a user input provided at a user input interface. In this way, an operator or individual may be able to define the desired value for each of the two or more image quality metrics.

In a more complex example, the desired quality indicator may comprise generating a desired quality indicator responsive to an identified anatomical region, e.g., for a specific anatomical region. In particular, a look-up table may correlate or cross-reference each of a plurality of anatomical regions to a desired quality indicator. In this way, the desired value for each image quality metric may differ or be defined by the identified anatomical region.

To carry out this more complex example, step 230 may comprise a sub-step of obtaining an anatomical region indicator that indicates an anatomical region; and a sub-step of processing the anatomical region indicator using a look-up table, which cross-references anatomical region indicators to desired quality indicators, to obtain the desired quality indicator.

In this approach, the anatomical region indicator may be defined by a user input provided at a user interface. As an alternative example, the anatomical region indicator may be obtained by processing the medical imaging data, e.g., using an object detection algorithm. Thus, step 230 may comprise processing the medical imaging data, using an object detection algorithm, to identify the anatomical region represented in the medical imaging data.

Suitable examples of object detection algorithms are well known in the art, including the YOLO algorithm, first set out by Redmon, Joseph, et al. "You only look once: Unified, real-time object detection." Proceedings of the IEEE conference on computer vision and pattern recognition. 2016 or anatomical segmentation techniques, such as those proposed by Zhu, Wentao, et al. "AnatomyNet: deep learning for fast and fully automated whole-volume segmentation of head and neck anatomy." Medical physics 46.2 (2019): 576-589.

Other examples for defining a desired value for a particular image quality metric may be employed.

For instance, a desired value for a resolution metric (of an ultrasound image) may be defined or computed based on characteristics of the ultrasound imaging procedure used to produce the ultrasound image, e.g., a centre frequency, active aperture size and pulse length information. The beam geometry may be used to define the near field, focal and far field region locations helping to know the ideal or optimal value of resolution at a particular spatial location or depth.

As another example, a desired value for an intensity contrast metric (of an ultrasound image) may be defined or computed from the acoustic characterization of the ultrasound transducer (used to produce the ultrasound image) and different configured transmit-receive schemes on different mediums (high attenuation medium, low attenuation medium, different scatterers distribution in medium/phantoms (sparse or densely populated scatterers.

The computer-implemented method 200 further comprises a step 240 of defining imaging parameters of the medical imaging system responsive to the measured value and the desired value for each of the two or more image quality metrics.

In a simple example, step 240 may make use of a look-up table that maps modifications or changes for the imaging parameters responsive to a difference between the measured value and the desired value for each of the two or more image quality metrics. For instance, a first imaging parameter may be changed in a first way responsive to a difference between the measured value and the desired value of a first image quality metric meeting one or more criteria.

In another complex example, step 240 makes use of an optimization algorithm (such as gradient descent algorithm) to control the modification or defining of the imaging parameters. In particular, step 240 may comprise iteratively modifying one or more imaging parameters to monitor a change in the difference between the measured value and the desired value for each of the two or more image quality metrics.

In some approaches, a measure of error (e.g., produced using a loss function or a cost function) is employed in step 240. The measure of error may quantify and combine the differences between the desired value and the measured value of each image quality metric. The optimization algorithm may aim to minimize or reduce the measure of error through iterative modifications. It will be appreciated that, in practice, the optimization algorithm does not need to fully optimize the imaging parameters, but may rather terminate when one or more termination criteria have been met, e.g., the measure of error is below a predetermined thresholds, no fewer than a predetermined number of iterative modifications have been made, and so on.

$$L = \sum_{i=1}^{N} (d_i - m_i) \qquad (1)$$

Equation (1) provides an example of a measure of error L that sums the difference between the desired value $d_i$ and the measured value $m_i$ of each image quality metric i. The number N represents the total number of image quality metrics.

Other suitable measures of error will be apparent to the skilled person, e.g., an approach that determines a product of the differences between the desired value $d_i$ and the measured value $m_i$ of each image quality metric i.

Of course, it will be appreciated that in performing step 240 using an optimization algorithm (or similar), that steps 210 and 220 may need to be iteratively repeated to update the measured value for each image quality metric responsive to the change in imaging parameters.

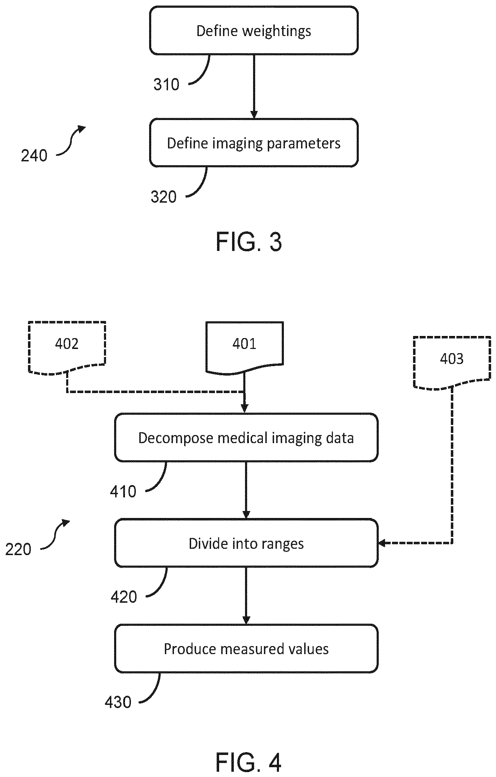

Fig. 3 illustrates another example approach for performing step 240 of defining imaging parameters.

In this approach, step 240 comprises a sub-step 310 of defining a weighting for each of the two or more image quality metrics responsive to the desired quality indicator, wherein the desired quality indicator indicates a desired value for each of the two or more image quality metrics. The weighting may be a value representing a relative importance or priority of the image quality metric.

Step 240 also comprises a sub-step 320 of defining imaging parameters responsive to the weighting and measured value for each of the two or more image quality metrics.

In this way, the desired quality indicator identifies a relative importance or weighting of each image quality metric. This approach allows for control over which image quality metrics are weighted or prioritized in the control of the imaging parameters. This approach can effectively prioritize certain image quality metrics over others.

Where the desired quality indicator is responsive to a user input, this effectively allows a user or individual to define or control which image quality metrics are prioritized over others. This allows greater flexibility and control for the individual to aid them in performing a clinical task of assessing the imaged subject using the medical imaging data.

Where the desired quality indicator is responsive to an anatomical region indicator, this allows different image quality metrics to be prioritized over others dependent upon the anatomical region of interest, e.g., the imaged anatomical region.

It is recognized that imaging and analysis of different anatomical regions is advantaged with different image quality metrics. More simply, different anatomies put different constraints on desired image quality metrics. For example, speckle is considered important in the case of liver imaging. Cardiac imaging poses strict requirements on contrast between the 4 chambers of the heart and its delineation. Imaging of bony structures such as spine demands very good resolution with better contrast, to precisely delineate sub-anatomies within the spine.

Suitable approaches for performing sub-step 320 will be apparent to the skilled person.

As a working example, sub-step 320 may make use of an optimization algorithm (such as gradient descent algorithm) and a weighted measure of error $L_W$ to control the modification or defining of the imaging parameters. In particular, step 240 may comprise iteratively modifying one or more imaging parameters and monitoring a change in a weighted measure of error to determine or select a next modification.

Thus, in some approach, a weighted measure of error (e.g., produced using a weighted loss function or a weighted cost function) is employed in step 240.

The optimization algorithm may aim to minimize or reduce the weighted measure of error through iterative modifications. It will be appreciated that, in practice, the optimization algorithm does not need to fully optimize the imaging parameters, but may rather terminate when one or more termination criteria have been met, e.g., the weighted measure of error is below a predetermined thresholds, no fewer than a predetermined number of iterative modifications have been made, and so on.

$$L_W = \sum_{i=1}^{N} w_i (d_i - m_i) \qquad (2)$$

Equation (2) provides an example of a weighted measure of error Lw that performs a weighted sum of the difference between the desired value $d_i$ and the measured value $m_i$ of each image quality metric i. The number N represents the total number of image quality metrics. The value $w_i$ represents a weighting for the image quality metric i. In this approach, the greater the weighting $w_i$, the greater the emphasis or priority of the corresponding image quality metric. Thus, the term $w_i(d_i - m_i)$ represents a weighted difference for a particular image quality metric.

Other suitable measures of error will be apparent to the skilled person, e.g., an approach that determines a product of the weighted differences between the desired value $d_i$ and the measured value $m_i$ of each image quality metric i.

It has previously been described how two or more image quality estimation algorithms are used to produce a measured value for each of a respective two or more image quality metrics. Suitable examples of image quality metrics, as well as image quality estimation algorithms, that may be employed in some embodiments are hereafter described.

Fig. 4 illustrates an example approach for performing step 220 of processing the medical imaging data using two or more image quality estimation algorithms to produce a measured value for each of a respective two or more image quality metrics.

In this approach, step 220 comprises a step 410 of processing the medical imaging data using a Fourier decomposition technique to produce a spatial frequency representation of the medical imaging data.

A wide number of Fourier decomposition techniques are known in the art, and are not described in detail for the sake of conciseness. In the context of the present disclosure, the Fourier decomposition technique effectively converts the medical imaging data into the spatial frequency domain. The spatial frequency representation of medical imaging data may be represented by a 2D image in which different pixels represent different spatial frequencies. It is conventional for high frequencies are mapped to be closer to the perimeter of the 2D image, with low frequencies being mapped to be closer to the center or origin of the 2D image.

In some examples, in step 410, the medical imaging data may further be processed (before undergoing decomposition) to identify the region within the medical imaging data comprising imaging data of the subject, i.e., representing (the appearance of) anatomical elements of the subject. The identified region may be the only region of the medical imaging data that is further processed.

This approach is particularly advantageous if the medical imaging data comprises additional information that do not represent the subject. For instance, some forms of ultrasound imaging data may comprise only a segment that represents the subject. Other medical imaging data types may comprise additional information (e.g., metadata) that does not directly represent an appearance of the subject.

To facilitate this procedure, step 410 may comprise obtaining a region mask 402 that identifies, within the medical imaging data, the region that represents (the appearance of) anatomical elements of the subject.

Step 220 may further comprise a step 420 of dividing the spatial frequency representation into at least two different frequency ranges to form a respective at least two partial spatial frequency representations.

In step 420 dividing the spatial frequency representation may comprise obtaining an anatomical indicator 403 that indicates an anatomical region; and defining the at least two different frequency ranges responsive to the anatomical indicator.

By way of example, the end frequencies for each frequency range (i.e., the upper and lower limits of each frequency range) may be preset for different anatomical regions. The end frequencies may be saved or defined in a look-up table (LUT). The anatomy selection may be performed either manually (i.e., responsive to a user input) or by processing the medical imaging data using an anatomy classification algorithm. The LUT may be consulted or referenced, to identify the respective end frequencies and decompose the image into the relevant frequency components.

This embodiment recognizes that different anatomies will have different end frequencies for each frequency range as a result of the underlying heterogeneity in the imaged medium (e.g., a liver is a homogeneous organ). In particular, because of the underlying cellular structure, different anatomical regions may only be representable using different ranges of frequencies. For instance, an ultrasound image of a liver may be best represented with only frequency coefficients mostly in the low frequency and mid-frequency zones of the 2D spatial frequency map. There are not many sharp transitions in a liver, as it is a relatively homogeneous organ, meaning that there is no or minimal high frequency detail to capture. As a counter example, breast tissue or a kidney is comparatively heterogeneous (e.g., compared to a liver) because of the wide variety of different types and sizes of cells that form such anatomical elements. The medical imaging data produced by medical imaging systems will behave differently based on the size of such microstructures and causes different spatial frequencies in any medical imaging data produced.

Put another way, low frequencies help in capturing the global aspect of the anatomy with higher frequencies helping in capturing the details. In more heterogeneous structure there are very fine details and those can only be captured by high spatial frequencies. However, this level of detail is not required for homogeneous structures, making it more relevant to focus upon lower frequencies.

In brief, more heterogeneous anatomical structures would benefit from having higher cutoff in terms of its spatial frequencies (to capture the detail) and more homogenous anatomical structure would benefit from having lower cutoffs in terms of its spatial frequencies (for better granularity at low frequencies, as high frequency information would not contain much data).

Step 220 may also comprise a step 430 of processing each partial spatial frequency using a different image quality estimation algorithm to produce the measured value for each of a respective two or more image quality metrics.

This approach recognizes that different image quality metrics can be produced from different spatial frequency ranges of information. For instance, high frequency components can be used to generate a speckle metric (which is most responsive to changes in high frequency). Similarly, low frequency components can be used to generate a contrast quality metric (which is most responsive to low frequency changes in imaging data). As another example, mid frequency components can be used to generate a resolution quality metric.

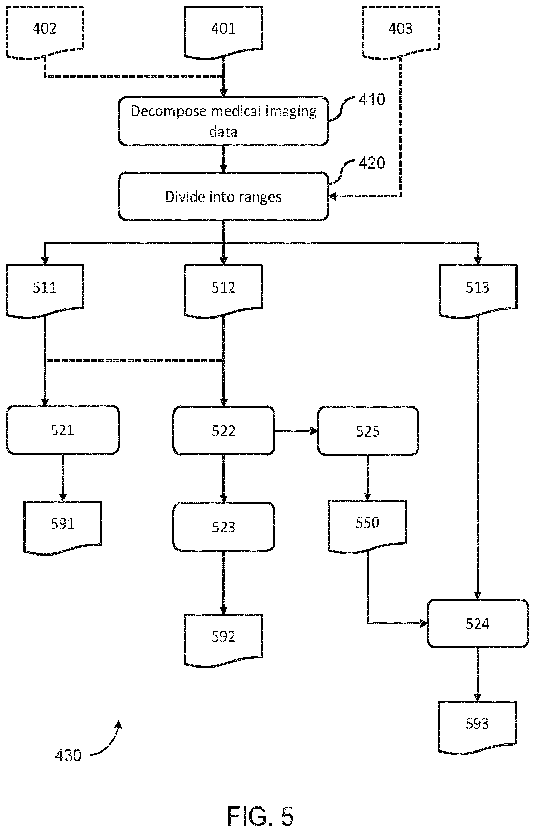

Fig. 5 is a flow diagram illustrating the computation of three image quality metrics, namely a speckle quality metric; a spatial or intensity contrast metric; and a resolution metric. These image quality metrics may be determined or calculated in some embodiments.

As previously explained, the medical imaging data 501 is processed, in a step 410, using a decomposition technique to produce spatial frequency representation of the medical imaging data. The spatial frequency representation is then divided, in a step 420, into at least two different frequency ranges to form a respective at least two partial spatial frequency representations. Image quality metrics are then produced using the at least two partial spatial frequency representations in a step 430, e.g., a low-frequency component 511, a mid-frequency component 512 and a high-frequency component 513.

The low-frequency component 511 can undergo reverse transformation to produce structure imaging data. The mid-frequency component 512 can undergo reverse transformation to produce sub-structure imaging data. The high-frequency component 513 can undergo reverse transformation to produce texture imaging data.

One example of an image quality metric is a contrast quality metric. Conventionally, contrast is measured by determining, in a manually selected region of interest, the ratio of average intensity values in the foreground region and average intensity values in the background region. One herein proposed approach for approximating a value for a contrast quality metric makes use of the low-frequency component 511 (e.g., structure imaging data produced therefrom). It is proposed to determine a value for two initial contrast quality metrics (i.e., two contrast factors) that are blended to produce a combined contrast quality metric (representing the complete approximate global contrast factor). The blending may be a weighted blending, e.g., where each contrast quality metric is weighted before the weighted contrast quality metrics are summed. This is also known as an $\alpha$-blending technique or a linear combination.

In this approach, values for a spatial contrast metric and an intensity contrast metric are determined and then combined in a process 521, to produce the value for the contrast quality metric 591.

For computing a value of a spatial contrast metric of ultrasound imaging data, the low-frequency component 511 (of the ultrasound imaging data) is transformed (e.g., using an inverse Fourier approach) to form the structure imaging data. This is then scaled at several factors to obtain a scale-space representation of the structure imaging data. The scaling factors may be powers of two to define a common standard. Then, the mean of the Laplacian of the structure imaging data, weighted by Gaussian weights from the spatial distribution centered at the focal zone of ultrasound during transmit, is used to compute the contrast value at each scale. The normalized weighted average of contrast values, with highest weightage to finest scale, is computed as the value for the spatial contrast metric. This metric also functions to capture a significant aspect of spatial intensity transitions that remains in the structure imaging data.

For computing a value of an intensity contrast metric, the low-frequency component 511 is transformed (e.g., using an inverse Fourier approach) to form the structure imaging data. This may be performed once for calculating both the spatial and the intensity contrast metrics. The structure imaging data is then segmented into different echogenicity zones, which may be divided into 5 classes, namely, hyper-echoic, anechoic, weak scattering, strong scattering and medium scattering. The contrast to noise ratio between these regions is then then computed to produce the intensity contrast metric. One example approach to calculating the contrast to noise ratio CNR is as follows:

$$CNR = \sum_{j=1}^{k-1} w_j * abs\left(\frac{\mu_j}{\sigma_j} - \frac{\mu_{j+1}}{\sigma_{j+1}}\right) \qquad (3)$$

where j is the j-th echogenicity zone, k is of total number of zones, $w_j$ is a weighting for the j-th echogenicity zone, $\mu_j$ is the mean intensity of the j-th echogenicity zone and $\sigma_j$ is the standard deviation of the intensity of the j-th echogenicity zone. The function abs($\cdot$) is the absolute function for calculating an absolute value, i.e., a magnitude.

The weightings $w_j$ may be selected to define a window function for different values of the mean intensities, e.g., to window across a known range of possible values for the mean intensity. Suitable windows include a Hann window or a Hamming window. The precise values of the weighting $w_j$ may, in some examples, depend upon the anatomy represented by the ultrasound imaging data. Approaches for identifying an anatomical structure represented in ultrasound imaging data have been previously disclosed.

Another example of an image quality metric 592 is a resolution quality metric. An approach for calculating a resolution quality metric for an ultrasound image, an example of medical imaging data, is hereafter described. The skilled person would be readily capable of using other forms of resolution calculation mechanisms for other types of medical imaging data.

In the context of ultrasound images, the resolution quality metric (which quantifies resolution) may measure the spread of a point in axial and lateral directions. The spread is estimated as width of the peak at half of its maximum (FWHM) value. This also corresponds to width at -6 dB level in log scale. To estimate resolution of an ultrasound image, it is possible to make a statistical estimate. An edge can be considered as a collection of multiple point scatterers tied together. One algorithm 523 for generating a resolution quality metric involves taking profiles, of reconstructed medical imaging data, along axial and lateral directions. The reconstructed medical imaging data is produced by transforming the combination of the low-frequency component 511 and the mid-frequency component 512 into the image domain, e.g., using a reverse Fourier transform, in a process 522.

In the algorithm 523, peaks are detected for each of the aforementioned lines between certain amplitude levels corresponding to the hyperechoic zone, which may be determined based on echogenicity values, from which the FWHM value is estimated. These computed values are then placed at the spatial location of the peaks, to create a map of axial resolution. Similarly, the profiles are taken along the lateral direction. This provides a map of lateral resolution. A weighted map may then be created, assuming gaussian distribution centered at the focus of the ultrasound transmit beam (focal zone in ultrasound). The axial resolution and lateral resolution map are then weighted using this weight matrix and a weighted mean value is obtained. These values correspond to the global axial and lateral resolution of a given ultrasound image, thereby each representing an example of a resolution quality metric. The values may be combined to produce a value for a combined resolution quality metric.

Another example of an image quality metric is a speckle quality metric 593. One example of a speckle quality metric 593 is a speckle variance, which is traditionally measured as the variance of a uniform patch that is selected manually. One herein proposed process 524 for approximating a speckle variance (to produce a measured value of the speckle quality metric) makes use of the high frequency component 513 of the medical imaging data.

For computing a value of the speckle quality metric, the high-frequency component 511 is transformed (e.g., using an inverse Fourier approach) to form the texture imaging data. The texture imaging data is divided into a $p \times q$ grid of patches, and the variance in every patch (that is void of edges) is computed. Edge pixels can be identified in a step 525 that processes reconstructed medical imaging data by transforming the combination of the low-frequency component 511 and the mid-frequency component 512 into the image domain, e.g., using a reverse Fourier transform, e.g., in the previously mentioned process 522. In this way, edges are identified, e.g., in the form of a mask 550, from the low frequency and the mid frequency components of the medical imaging data image. The edges may, for instance, be identified in step 525 using an edge detector process (such as the Canny edge detector) to capture edges at multiple scales. In step 524, all the valid patches (i.e., patches void of edges) are assigned Gaussian weights with focus at the center. The spatially weighted average of the variance from all the valid patches, with higher weights at ultrasound focal region, is computed as the value of the speckle quality metric.

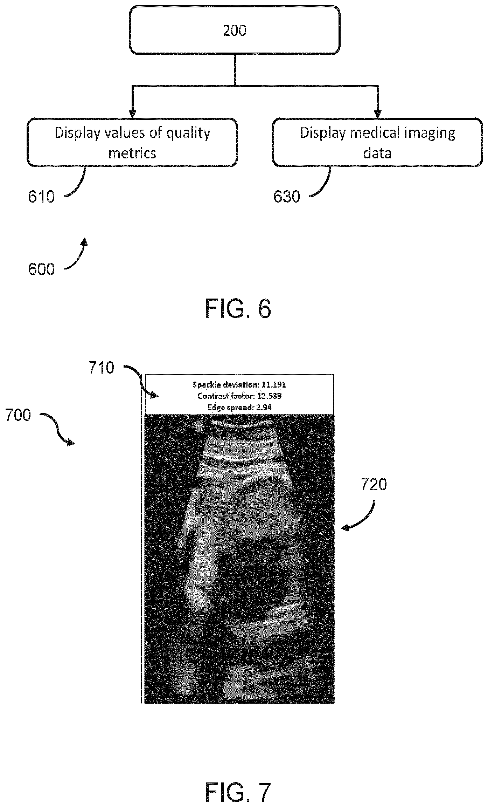

Fig. 6 is a flowchart illustrating a further method 600, which may be carried out by the processing system.

The method 600 comprises performing any previously described method 200 for defining imaging parameters of a medical imaging system.

The method 600 further comprises a step 610 of controlling a user output interface to provide a visual representation of the measured value of each of the two or more image quality metrics.

The method 600 may further comprise a step 620 of controlling the user output interface to provide a visual representation of the medical imaging data.

Fig. 7 provides an example visual representation 700 of both the measured value of each of the two or more image quality metrics and the medical imaging data. Thus, the visual representation comprises a first portion 710 that visually represents the measured value of each of the two or more image quality metrics, as well as a second portion 720 that visually represents the medical imaging data.

In the illustrated example, the medical imaging data comprises ultrasound imaging data of a fetal heart. More specifically, the medical imaging data comprises one or more 2D ultrasound images.

The skilled person would be readily capable of developing a processing system for carrying out any herein described method. Thus, each step of the flow chart may represent a different action performed by a processing system, and may be performed by a respective module of the processing system.

Embodiments may therefore make use of a processing system. The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

There is also proposed a system comprising the processing system previously disclosed and a medical imaging system. Preferably, the medical imaging system comprises an ultrasound imaging system. The ultrasound imaging system may comprise an ultrasound probe for generating, as the medical imaging data, ultrasound imaging data.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method.

There is also proposed a non-transitory storage medium that stores or carries a computer program or computer code that, when executed by a processing system, causes the processing system to carry out any herein described method.

In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. The mere fact that certain measures are recited in mutually different dependent claims does not indicate that a combination of these measures cannot be used to advantage.

In the claims, the word "comprising" does not exclude other elements or steps, and the indefinite article "a" or "an" does not exclude a plurality. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

A single processor or other unit may fulfill the functions of several items recited in the claims. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. A computer-implemented method (2000 for defining imaging parameters of a medical imaging system (110), the computer-implemented method comprising:

   obtaining (210), from the medical imaging system, medical imaging data of a subject (190);
   processing (220) the medical imaging data using two or more image quality estimation algorithms (521, 523, 524) to produce a measured value for each of a respective two or more image quality metrics;
   obtaining (230) a desired quality indicator indicating a desired value for each of the two or more image quality metrics (591, 592, 593); and
   defining (240) imaging parameters of the medical imaging system (110) responsive to the measured value and the desired value for each of the two or more image quality metrics.

2. The computer-implemented method of claim 1, wherein the defining imaging parameters comprises:

   defining (310) a weighting for each of the two or more image quality metrics responsive to the desired quality indicator; and
   defining (320) imaging parameters responsive to the weighting and measured value for each of the two or more image quality metrics.

3. The computer-implemented method of claim 1 or 2, wherein the obtaining the desired quality indicator comprises obtaining the desired quality indicator from a user input provided at a user input interface.

4. The computer-implemented method of any of claims 1 to 3, wherein the obtaining the desired quality indicator comprises:

   obtaining an anatomical region indicator that indicates an anatomical region; and

processing the anatomical region indicator using a look-up table, which cross-references anatomical region indicators to desired quality indicators, to obtain the desired quality indicator.

5. The computer-implemented method of claim 4, wherein the obtaining the anatomical region indicator comprises processing the medical imaging data, using an object detection algorithm, to identify the anatomical region represented in the medical imaging data.

6. The computer-implemented method of any of claims 1 to 5, wherein the medical imaging data comprises ultrasound imaging data.

7. The computer-implemented method of claim 6, wherein the medical imaging data comprises one or more 2D ultrasound images.

8. The computer-implemented method of any of claims 1 to 7, wherein the two or more image quality metrics comprise at least two of the following: a speckle quality metric; a spatial or intensity contrast metric; and/or a resolution metric.

9. The computer-implemented method of any of claims 1 to 8, wherein each image quality estimation algorithm comprises:

   processing (410) the medical imaging data (401) using a Fourier decomposition technique to produce a spatial frequency representation of the medical imaging data;
   dividing (420) the spatial frequency representation into at least two different frequency ranges to form a respective at least two partial spatial frequency representations (511, 512, 513); and
   processing (430) each partial spatial frequency using a different image quality estimation algorithm to produce the measured value for each of a respective two or more image quality metrics.

10. The computer-implemented method of claim 9, wherein dividing the spatial frequency representation comprises:

   obtaining an anatomical indicator that indicates an anatomical region; and
   defining the at least two different frequency ranges responsive to the anatomical indicator.

11. The computer-implemented method of any of claims 1 to 10, further comprising controlling a user output interface to provide a visual representation of the measured value of each of the two or more image quality metrics.

12. A computer program product comprising computer program code means which, when executed on a computing device having a processing system, cause the processing system to perform all of the steps of the method according to any of claims 1 to 11.

13. A processing system (120) for defining imaging parameters of a medical imaging system (110), the processing system being configured to:

   obtain (210), from the medical imaging system (110), medical imaging data of a subject;
   process (220) the medical imaging data using two or more image quality estimation algorithms (521, 523, 524) to produce a measured value for each of a respective two or more image quality metrics;
   obtain (230) a desired quality indicator indicating a desired value for each of the two or more image quality metrics; and
   define (240) imaging parameters of the medical imaging system (110) responsive to the measured value and the desired value for each of the two or more image quality metrics.

14. The processing system of claim 13, wherein the processing system is configured to define imaging parameters by:

   defining a weighting for each of the two or more image quality metrics responsive to the desired quality indicator; and
   defining imaging parameters responsive to the weighting and measured value for each of the two or more image quality metrics.

15. A system (10) comprising:

the processing system (120) of any of claims 13 or 14; and

an ultrasound imaging system (110), configured to function as the medical imaging system, wherein the ultrasound imaging system comprises an ultrasound probe configured to generate ultrasound imaging data of the subject to function as the medical imaging data of the subject.

FIG. 1

FIG. 2

Define weightings

310

240

Define imaging parameters

320

# FIG. 3

402

401

403

Decompose medical imaging data

410

220

Divide into ranges

420

Produce measured values

430

# FIG. 4

FIG. 5

200

Display values of quality metrics

Display medical imaging data

610

630

600

## FIG. 6

710

700

Speckle deviation: 11.191
Contrast factor: 12.539
Edge spread: 2.94

720

## FIG. 7

Europäisches
Patentamt

European
Patent Office

Office européen
des brevets

# EUROPEAN SEARCH REPORT

**Application Number**

EP 24 15 6086

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 4 285 825 A1 (KONINKLIJKE PHILIPS NV [NL]) 6 December 2023 (2023-12-06) * paragraphs [0009] - [0043], [0053] - [0077], [0136] - [0140]; claims; figures * | 1-15 | INV. A61B8/00 A61B8/08 |
| A | WO 2023/061910 A1 (KONINKLIJKE PHILIPS NV [NL]) 20 April 2023 (2023-04-20) * claims; figures * | 1-15 | |
| A | US 2021/169455 A1 (ANNANGI PAVAN KUMAR V [IN] ET AL) 10 June 2021 (2021-06-10) * paragraph [0017]; claims; figures * | 1-15 | |
| A | US 2023/148998 A1 (OWEN CYNTHIA A [US] ET AL) 18 May 2023 (2023-05-18) * paragraphs [0009] - [0016]; claims; figures * | 1-15 | |
| A | US 2010/240992 A1 (HAO XIAOHUI [US]) 23 September 2010 (2010-09-23) * paragraph [0023] * | 1-15 | **TECHNICAL FIELDS SEARCHED (IPC)** A61B |

The present search report has been drawn up for all claims

| Place of search | Date of completion of the search | Examiner |
|---|---|---|
| Munich | 28 June 2024 | Mundakapadam, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 4 599 771 A1**

## ANNEX TO THE EUROPEAN SEARCH REPORT
## ON EUROPEAN PATENT APPLICATION NO.

EP 24 15 6086

This annex lists the patent family members relating to the patent documents cited in  the above-mentioned European search report.
The members are as contained in the European Patent Office EDP file on
The European Patent Office is in no way liable for these particulars which are merely given for the purpose of information.

28-06-2024

| Patent document cited in search report | | Publication date | Patent family member(s) | | Publication date |
|---|---|---|---|---|---|
| EP 4285825 | A1 | 06-12-2023 | EP | 4285825 A1 | 06-12-2023 |
| | | | WO | 2023232731 A1 | 07-12-2023 |
| WO 2023061910 | A1 | 20-04-2023 | NONE | | |
| US 2021169455 | A1 | 10-06-2021 | CN | 112890853 A | 04-06-2021 |
| | | | US | 2021169455 A1 | 10-06-2021 |
| US 2023148998 | A1 | 18-05-2023 | CN | 116115256 A | 16-05-2023 |
| | | | US | 2023148998 A1 | 18-05-2023 |
| US 2010240992 | A1 | 23-09-2010 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ZHU, WENTAO et al.** AnatomyNet: deep learning for fast and fully automated whole-volume segmentation of head and neck anatomy.. *Medical physics*, 2019, vol. 46.2, 576-589